(19) Europäisches Patentamt
European Patent Office
Office européen des brevets

(11) Publication number: 0 213 391
A2

(12) # EUROPEAN PATENT APPLICATION

(21) Application number: 86110378.6

(22) Date of filing: 20.05.83

(51) Int. Cl.⁴: A 61 K 39/00
A 61 K 39/385, A 61 K 37/38
C 07 K 3/08

(30) Priority: 04.03.83 US 472190

(43) Date of publication of application:
11.03.87 Bulletin 87/11

(84) Designated Contracting States:
AT BE CH DE FR IT LI LU NL SE

(60) Publication number of the earlier application in accordance with Art. 76 EPC: 0 117 934

(71) Applicant: The Ohio State University
200 Administration Building 190 North Oval Mall
Columbus Ohio 43210(US)

(72) Inventor: Stevens, Vernon Cecil
5481 River Forest Road
Dublin, Ohio 43017(US)

(74) Representative: Cole, David John
Executive Liaison Services 54 Templemere
Weybridge Surrey KT13 9PB(GB)

(54) **Antigenic modification of polypeptides.**

(57) Modified polypeptides capable of provoking the formation of antibodies in an animal may be produced by forming a linear polymer of polypeptide fragments, each having a molecular structure similar to a fragment of the protein to which antibodies are to be provoked. Such linear polymers can be made more immunogenic than the proteins to which they are related without introducing undesirable extraneous materials into the animal being treated, but have reproducable immunogenic properties. Protein which are not endogenous or immunogenic to an animal can be chemically modified so as to make them more immunogenic. Also, modified antigens useful for fertility control can be produced by chemical modification of zona pellucida or sperm antigens. These modified polypeptides, antigens and modified antigens are desirably administered in the form of a vaccine having a vehicle comprising a mixture of mannide monooleate with Squalene and/or Squalene.

EP 0 213 391 A2

# ANTIGENIC MODIFICATION OF POLYPEPTIDES

This invention relates to antigenic modification of polypeptides. More particularly, this invention relates to specific techniques for modification of polypeptides, to antigens for provoking the formation, by the immune system of an animal, of antibodies to exogenous proteins e.g. proteins found in viruses and other pathogenic organisms, and to processes for fertility control using chemically-modified antigens.

In our British patent No. 1,567,764 and in our U.S. patent No. 4,302,386 (the disclosures of which are herein incorporated by reference) it is disclosed that hormones or other proteins found naturally in an animal can be chemically modified outside the body of the animal (a term which is used herein to include human beings) so that, when injected into the animal, the modified hormones or other proteins provoke the formation of antibodies which react not only with the chemically-modified hormone or other protein but also with the unmodified hormone or other protein found naturally in the animal, thereby reducing the level of the natural hormone or other protein in the body of the animal. Instead of using the entire hormone or other protein, the aforementioned patents also disclose the use of natural or synthetic fragments of such proteins in this so-called "isoimmunization" technique.

The aforementioned patents are mainly concerned with the application of their isoimmunoization technique in controlling fertility, so that the protein which is modified is a reproductive hormone, such as Follicle Stimulating Hormone (FSH), Leutinising Hormone (LH), Human Placental Lactogen (HPL), Human Prolactin and Human Chorionic Gonadotropin (HCG), or a peptide having an amino acid sequence corresponding to a part of one of these hormones. However, the aforementioned patents also disclose the applicability of the isoimmunization technique to other protein hormones, for example:

1. Gastrin, for the treatment of Zollinger-Ellison Syndrome;
2. Angiotension II, for the treatment of hypertension;
3. Growth hormone and somatomedian, for the treatment of diabetes and associated micro and macro vascular diseases;
4. Parathyroid hormone for the treatment of kidney stones;
5. Insulin and glucagon, for the treatment of hyperinsulinoma;
6. Thyroid stimulating hormone, for the treatment of hyperthyroidism;
7. Secretin, for the treatment of irritable bowel syndrome.

The aforementioned patents also disclose the use of modified polypeptides derived from chorionic gonadotropin, LH or FSH, or a fragment thereof, for use in treating certain carcinomas.

The main technique of chemical modification disclosed in the aforementioned patents is coupling of the hormone, other than protein or fragment thereof to a large "carrier" molecule such as diphtheria toxoid, tetanus toxoid or a synthetic polymer; these carrier molecules are not endogenous to the animal to be treated. The aforementioned patents also describe polymerization of the hormone, other protein, or fragment thereof by reaction with a bifunctional organic reagent (for example a bi-functional imidoester such as dimethyl adipimidate, dimethyl suberimidate or diethyl malonimidate) or dimerization by oxidation of a thiol group to form a disulfide bridge. All these modes of chemical modification have disadvantages. Dimerization of the hormone, other protein or fragment via a disulfide bridge does not introduce exogenous material into the animal being treated but, since the chemically-modified antigen administered to the animal is only a dimer of a hormone, other protein or fragment which is not itself immunogenic to the animal, such dimers are rarely successful in provoking useful levels of antibodies. The modified polypeptides produced using carriers contain a very high proportion of non-endogenous material, and will usually provoke the formation of substantial quantities of antibodies to the carrier as well as to the hormone, other protein or fragment thereof. Although the formation of antibodies to the carrier may be sometimes be useful (for example, a vaccine based upon a HCG fragment coupled to diphtheria toxoid and intended for fertility control has the incidental advantage of also conferring protection against diphtheria), there are many occasions upon which it is not desirable to provoke the formation of relatively large quantities of antibodies to the carrier; for example, if one wishes to use a vaccine containing a modified polypeptide to treat a patient with a carcinoma or a serious viral infection, it may be desirable to avoid overstraining the patient's immune system by challenging it not only with the hormone, other protein or fragment thereof to which antibodies are desired, but also with the carrier.

In theory, perhaps the most promising of the modification techniques disclosed in the aforementioned patents is polymerization of the hormone, other protein or fragment thereof by reaction with a bifunctional reagent. This technique has, in theory, the advantages of introducing a relatively small proportion of non-endogenous material into the animal being treated (and even this relatively small proportion of non-endogenous material can be chosen so that it is not strongly

immunogenic), while still providing a modified polypeptide large enough to be strongly immunogenic. Unfortunately, experiments have proved that straightforward application of the bifunctional organic reagent polymerization technique to most hormones, other proteins or fragments thereof of practical interest produces very complicated mixtures of modified polypeptides having correspondingly complicated immunogenic properties. Furthermore, the immunogenic properties of the polymerized polypeptides thus produced are not readily reproduceable, but such reproduceability is essential in any material intended for pharmaceutical use, since the necessary tests of safety and efficiency cannot be performed on non-reproduceable material.

We have now found (though this knowledge is not in the published literature) that the reason for the very complicated immunogenic properties and the lack of reproduceability present in polymers produced by the bifunctional organic reagent polymerization technique is that, notwithstanding the use of a bifunctional reagent, extensive cross-linking of the hormone, other protein fragment thereof occurs, such cross-linking presumably being due to the presence of free amino, thiol, carboxyl and perhaps other groups (the exact groups involved depending of course upon which groups the bifunctional organic reagent is designed to react with) at non-terminal positions on the hormone, protein or fragment thereof. Such cross-linking produces branching and three-dimensional structure in the resultant polymers. Not only does the relatively random cross-linking thus produced render the structure of the polymers themselves unpredictable and non-reproduceable, but such cross-linking may well alter the tertiary structure and shape of the hormone, other protein or fragment thereof being polymerized, thus affecting its immunogenic properties.

Accordingly, we have concluded that to produce useful modified polypeptides by the bifunctional organic reagent polymerization technique, it is essential to operate in such a way that coupling of the polypeptide fragments being polymerized occurs only at or near the terminals of the fragments, thus producing a true linear polymer substantially free of non-linear polymers of the fragments.

It has also been found that the isoimmunization technique described in the aforementioned patent can usefully be extended to proteins which are not endogenous nor substantially immunogenic to the animal to be treated. Finally, it has been found that modified antigens for use in fertility control can be produced by chemical modification of zona pellucida or sperm antigens.

Accordingly, in one aspect this invention defines a modified polypeptide for provoking the formation, in the body of an animal, of antibodies to a protein, the

modified polypeptide being characterized in that it comprises a linear polymer of polypeptide fragments, each of the fragments, in its monomeric form, being substantially non-immunogenic to the animal and having a molecular structure similar to a fragment of the protein to which antibodies are to be provoked, the linear polymer, after administration into the body of the animal, having a greater capacity to provoke the formation of the antibodies than the protein, the linear polymer being substantially free of non-linear polymers of the fragments.

In another aspect, this invention provides a method for producing a linear polymeric polypeptide for provoking the formation, in the body of an animal, of antibodies to one or more proteins which is substantially non-immunogenic to the animal, the method being characterized by:

(a) procuring a first peptide having a molecular structure similar to a fragment of the or one of the proteins;

(b) reacting this first peptide with a bifunctional coupling reagent while the first peptide is in a form having only a single site capable of reacting with the coupling reagent, this site being at or adjacent one of the termini of the first peptide, thereby causing one of the functional groups of the coupling reagent to react with the one site on the peptide;

(c) reacting the product of step (b) with a second peptide having a molecular structure similar to a fragment of the or one of the proteins while the second peptide is in a form having only a single site capable of reacting with the other functional group of the coupling reagent, this site being at or adjacent one of the termini of the second peptide, thereby forming a dimeric peptide wherein the first and second peptide are interconnencted via a residue of the coupling reagent;

(d) reacting the resultant peptide with a bifunctional coupling reagent while the preptide is in a form having only a single site capable of reacting with the coupling reagent, this site being at or adjacent one of the termini of the peptide, thereby causing one of the functional groups of the coupling reagent to react with the one site on the peptide;

(e) reacting the product of step (d) with a further peptide having a molecular structure similar to a fragment of the or one of the proteins while this further peptide is in a form having only a single site capable of reacting with the other functional group of the coupling reagent, this site being at or adjacent one of the termini of the further peptide, thereby forming a polymeric peptide wherein the first, second and further peptides are interconnected via residues of the coupling reagent;

(f) repeating steps (d) and (e) until the desired polymer length has been achieved.

In another aspect, this invention provides a method for producing a modified polypeptide for provoking the formation, in the body of an animal, of antibodies to a protein which is substantially non-immunogenic to the animal, the method being characterized by:

a. procuring a first peptide having a molecular structure similar to a fragment of the protein, the first peptide not having an unblocked thiol group and having an unblocked amino group only at its N-terminal but no other unblocked amino group;

b. reacting the first peptide with an amino group activating agent, thereby producing an activated amino group at the N-terminal of the first peptide;

c. reacting the activated first peptide with a second peptide having a molecular structure similar to a fragment of the protein, the second peptide having a C-terminal cysteine bearing an unblocked thiol group but not having any other unblocked thiol groups, thereby coupling the N-terminal of the first peptide to the C-terminal of the second peptide;

d. reacting the resultant compound in a form having an unblocked amino group at its N-terminal but no other unblocked amino groups, and no unblocked thiol group, with an amino-group activating agent, thereby producing an activated amino-group at the N-terminal of the resultant compound;

e. reacting the activated compound produced in step (d) with a further peptide having a molecular structure similar to a fragment of the protein, this further peptide having a C-terminal cysteine bearing an unblocked thiol group, but not having any other unblocked thiol groups, thereby coupling the activated N-terminal of the reactivated compound produced in step (d) to the C-terminal of the further peptide; and

f. repeating steps (d) and (e) until the desired polymer length has been achieved.

In another aspect, this invention provides an antigen for provoking the formation, in the body of an animal, of antibodies to a protein which is not endogenous nor substantially immunogenic to the animal, characterized in that the antigen comprises the protein, or a peptide having a sequence corresponding to at least part of the sequence of the protein, which protein or peptide has been

chemically modified outside the body of the animal, the antigen having a greater capacity to provoke the formation of the antibodies than the protein in its unmodified form.

In another aspect, this invention provides a process for preparing an antigen of the invention, which process is characterized by:

procuring the protein which is not endogenous or immunogenic to the animal, or the peptide having a sequence corresponding to at least part of the sequence of the protein; and

chemically modifying the protein or peptide outside the body of the animal, thereby producing the antigen according to claim 3.

In another aspect, this invention provides a modified antigen for use in fertility control in an animal characterized in that it comprises an antigen derived from the zona pellucida or from sperm, or a peptide having a sequence corresponding to at least part of the sequence of such a zona pellucida or sperm antigen, which antigen or peptide has been chemically modified outside the body of the animal, the modified antigen, after administration into the body of the animal, having a greater capacity to provoke the formation of antibodies than the unmodified antigen from which it is derived.

Finally, this invention provides a vaccine for provoking the formation, in the body of an animal, of antibodies to a protein, this vaccine being characterized in that it comprises a modified polypeptide, antigen or modified antigen of the invention and a vechicle comprising a mixture of mannide monooleate with squalane and/or squalene.

As already mentioned, the linear polymeric modified polypeptides of the invention comprise inert polymers of polypeptide fragments substantially free of non-linear polymers. In saying that the polypeptide fragments used in the linear polymeric polypeptide of the invention have a molecular structure similar to a fragment of the protein to which antibodies are to be provoked, we do not necessarily imply that the entire amino acid sequence of each fragment must correspond exactly to part of the protein to which antibodies are to be provoked; for example, in certain cases certain substitutions of amino acids may be possible without affecting the immunogenic character of the fragment. For example, our aforementioned U.S. patent No. 4,302,386 describes a polypeptide, designated Structure (IX), which is notionally derived from the beta subunit of HCG but in which the cysteine residue at the 110 position is replaced by alpha-aminobutyric acid. In particular, when it is desired to provoke the formation of antibodies to an

endogenous protein, the polypeptide fragments used to form the appropriate modified polypeptide of the invention must have a molecular structure similar to a fragment of the endogenous protein which antibodies are to be provoked, this does not exclude the possibility that such fragments might actually be derived from a protein in a different species, since many proteins are either identical between species or differ from one another so little in amino acid sequence that considerable cross-reactivity exists between antibodies to the corresponding proteins in the two species. For example, as mentioned below, zona pellucida enzymes from a pig will, when injected into humans, produce antibodies which display considerably activity against human zona antigens. Accordingly, for example, if one wishes to form a modified polypeptide for provoking the formation of antibodies, in humans, to zona pellucida antigens, appropriate polypeptide fragments may be prepared from zona pellucida antigens of pigs. Also, the fragments used in the linear polymeric polypeptides of the invention may incorporate sequences of amino acids having no counterpart in the sequence of the protein from which the fragment is notionally derived. Again, for example, our aforementioned U.S. patent No. 4,302,386 describes certain polypeptide fragments, designated Structure (IV), (VIII), (IX), (X) and (XIV) which are notionally derived from the beta subunit of HCG but which incorporate spacer sequences comprising multiple proline residues.

It may at first appear surprising that a linear polymer of a polypeptide, the monomeric form of which is effectively non-immunogenic to an animal, can be immunogenic to the same animal. It is believed (though the invention is in no way limited by this belief) that the increase in immunogenicity upon polymerization is due to the increase in physical size of the molecule, which enables the molecule to be recognized much more easily by the animal's immune system. It can be shown that at least some monomeric polypeptides are very weakly immunogenic and cause the animal's immune system to produce detectable quantities of antibodies, which quantities, however are much too small to be effective. Immune systems are not well-adapted to recognize molecules as small as the small polypeptides when the polypeptides are present in polymeric form.

It will be apparent to those skilled in the art that the polypeptide fragments used in the linear polymeric polypeptides of the invention may be either natural (i.e. derived from natural proteins) or may be produced synthetically. Obviously, a synthetic polypeptide will perform in the same manner as a naturally occuring one in as much as the immune system of the animal to which the modified polypeptide is administered will react in exactly the same way to both. Also the fragments within

each linear polymer may be the same or different and need not necessarily be derived from the same protein.

The linear polymeric polypeptides of the invention may be used to provoke the formation of antibodies to both endogenous and non-endogenous proteins by using appropriate polypeptide fragments to form the polymers. (The term "endogenous" is used herein to denote a protein which is native to the species to be treated, regardless of whether the antigen is endogenous to the particular individual animal being treated. Thus, for example, for present purposes a porcine sperm antigen is regarded as being endogenous to a sow even though obviously such a sperm antigen will not normally be present in the body of a sow.) Further details of the use of non-endogenous proteins fragments of which may be utilized in the linear polymeric polypeptides of the invention is given below in connection with the discussion of the antigens of the invention. As regards endogenous proteins, fragments of which may be used to form the linear polymeric polypeptides of the invention, in general it may be stated that such endogenous proteins may include any of the proteins mentioned in our aforementioned U.S. patent No. 4,302,386, as described above. Incidentally, it should be noted that the use of the linear polymeric polypeptides of the invention based upon growth hormone and/or somatomedian is not confined to diabetic patients. Thus, the linear polymeric polypeptides of the invention based upon these two hormones may be used to treat non-diabetic patients, such as persons suffering from acromegaly, who have excessive levels of growth hormone and/or somatomedian.

Particularly preferred linear polymeric polypeptides of the invention are those formed from fragments having a molecular structure similar to a fragment of Human Chorionic Gonadotropin, and in particular similar to a fragment of the beta-subunit thereof. Two particularly preferred fragments for use in the linear polymeric polypeptides of the invention are:

Asp-Asp-Pro-Arg-Phe-Gln-Asp-Ser-Ser-Ser-Ser-Lys-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-Cys (hereinafter designated fragment A); and

Asp-His-Pro-Leu-Thr-Cys-Asp-Asp-Pro-Arg-Phe-Gln-Asp-Ser-Ser-Ser-Ser-Lys-Ala-Pro-Pro-Pro-Ser-Leu-Pro-Ser-Pro-Ser-Arg-Leu-Pro-Gyl-Pro-Ser-Asp-Thr-Pro-Ile-Leu-Pro-Gln-Cys.

Other HCG-derived fragments usable in the linear polymeric polypeptides of the invention include those of Structure (II)-(VIII), (VIIIa) and (IX)-(XIV) as set forth below, further details of the immunological nature of these fragments being given in the aforementioned U.S. patent No. 4,302,386:

Asp-Asp-Pro-Arg-Phe-Gln-Asp-Ser-Ser-Ser-Ser-Lys-Ala-Pro-Pro-
Pro-Ser-Leu-Pro-Ser-Pro—Arg-Leu-Pro-Gly-Pro-Ser* -Asp-Thr-
Pro-Ile-Leu-Pro-Gln

Structure (II)

Gln-Asp-Ser-Ser-Ser-Ser-Lys-Ala-Pro-Pro-Pro-Ser-Leu-Pro-Ser-
Pro-Ser-Arg-Leu-Pro-Gly-Pro-Ser-Asp-Thr-Pro-Ile-Leu-Pro-Gln

Structure (III)

Cys-Pro-Pro-Pro-Pro-Pro-Pro-Ser-Asp-Thr-Pro-Ile-Leu-Pro-
Gln

Structure (IV)

Asp-Asp-Pro-Arg-Phe-Gln-Asp-Ser-Pro-Pro-Pro-Pro-Pro-Pro-Cys

Structure (V)

Phe-Gln-Asp-Ser-Ser-Ser-Ser-Lys-Ala-Pro-Pro-Pro-Ser-Leu-Pro-
Ser-Pro-Ser-Arg-Leu-Pro-Gly-Pro-Ser-Asp-Thr-Pro-Ile-Leu-
Pro-Gln

Structure (VI)

Asp-Asp-Pro-Arg-Phe-Gln-Asp-Ser-Ser-Ser-Ser-Lys-Ala-Pro-Pro-
Pro-Ser-Leu-Pro-Ser

Structure (VII)

Asp-Asp-Pro-Arg-Phe-Gln-Asp-Ser-Pro-Pro-Pro-Cys-Pro-Pro-Pro-
Ser-Asp-Thr-Pro-Ile-Leu-Pro-Gln

Structure (VIII)

Asp-Asp-Pro-Arg-Phe-Gln-Asp-Ser-Pro-Pro-Pro-Pro-Pro-Pro-Cys-
Pro-Pro-Pro-Pro-Pro-Pro-Ser-Asp-Thr-Pro-Ile-Leu-Pro-Gln

Structure (VIIIa)

Asp-His-Pro-Leu-Thr-Aba-Asp-Asp-Pro-Arg-Phe-Gln-Asp-Ser-Ser-Ser-Ser-Lys-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-Pro-Pro-Pro-Pro-Pro-Pro-Cys

Structure (IX)

Asp-Asp-Pro-Arg-Phe-Gln-Asp-Ser-Ser-Ser-Ser-Lys-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-Pro-Pro-Pro-Pro-Pro-Pro-Cys

Structure (X)

Asp-Asp-Pro-Arg-Phe-Gln-Asp-Ser-Ser-Ser-Ser-Lys-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-Cys

Structure (XI)

Thr-Cys-Asp-Asp-Pro-Arg-Phe-Gln-Asp-Ser-Ser-Ser-Ser-Lys-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln

Structure (XII)

Asp-His-Pro-Leu-Thr-Aba-Asp-Asp-Pro-Arg-Phe-Gln-Asp-Ser-Ser-Ser-Ser-Lys-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-Cys

Structure (XIII)

Cys-Pro-Pro-Pro-Pro-Pro-Pro-Asp-Asp-Pro-Arg-Phe-Gln-Asp-Ser-Ser-Ser-Ser-Lys-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln

Structure (XIV)

Obviously, if it is desired to use one of the above peptides which lacks a C-terminal cysteine as a second or later fragment in preparing the linear polymeric peptides of the invention, it will be necessary to add a C-terminal cysteine to the peptide; appropriate methods for doing so are of course well known to those skilled in the field of polypeptide synthesis. Also, some of the above peptides will of course require blocking of non-terminal amino and/or thio groups before use.

The linear polymeric polypeptides of the invention based upon HCG-derived fragments are usable in exactly the same manner as the corresponding modified polypeptides in which the same fragments are coupled to carriers, as described in our aforementioned U.S. patent No. 4,320,386. Moreoever, since this patent was written, further discoveries have been made concerning the association between HCG and immunologically similar materials and certain carcinomas. It appears (though the invention is in no way limited by this belief) that certain carcinomas exude chorionic gonadotropin or an immunologically-similar material on their surfaces, thereby presenting to the immune system of the host animal a surface, which, superficially, appears to be formed of material endogenous to the host animal, and which is thus relatively non-immunogenic. Because of this known association between certain carcinomas and chorionic gonadotropin or chorionic gonadotropin-like materials, the HCG-derived linear polymeric polypeptides of the invention are useful for the treatment of HCG and chorionic gonadotropin-association carcinomas. The same linear polymeric polypeptides of the invention are also of course useful for fertility control, as described in the aforementioned U.S. patent.

The fragments used in the linear polymeric polypeptides of the invention may also include fragments of the zona pellucida and sperm antigens discussed in detail below.

As already mentioned, the linear polymeric peptides of the invention are produced by polymerizing fragments of the protein to which antibodies are to be provoked rather than the entire protein itself. This use of fragments rather than an entire proteins has important advantages (and similar advantages are securred by the use of fragments in the antigens and modified antigens of the invention discussed in detail below). It is well recognized by those skilled in immunology (see e.g. W.R. Jones, "Immunological Fertility Regulation", Blackwell Scientific Publications, Victoria, Australia (1982) (the entire disclosure of this work is herein incorporated by reference), pages 11 *et seq.*) that one of the greatest potential hazards of a vaccine, especially a contraceptive vaccine, is cross-reactivity with non-target antigens, producing what is essentially an artificially-induced autoimmune disease capable of causing immunopathological lesions in, and/or loss of function of, the tisues carrying the cross-reactive substances. Two possible mechanisums for such cross-reactivity are:

(a) Presence of shared antigenic determinants; a complex protein may contain components (amino-acid sequences) identical to those present in a non-target antigens;

(b) steric overlap between non-identical but structurally related parts of the protein and non-target antigens.

Obviously, the changes posed by both these modes of cross-reactivity may be lessened by using, in the linear polymeric polypeptides, antigens and modified antigens of the invention, a fragment of a complex protein rather than the whole protein. Since the fragment has a simpler sequence than the protein from which it is derived, there is less chance of shared antigenic dterminants or steric overlap with non-target proteins. In particular, cross-reactions can often be avoided by using fragments derived from a portion of the target protein (i.e. the protein to which antibodies are to be provoked) which is not similar in sequence to the non-target but cross-reactive protein. In particular, as described in our aforementioned U.S. patent No. 4,320386, one of the major problems in provoking antibodies to HCG is cross-reactivity of HCG antibodies with LH, this cross-reactivity being at least largely due to identity of amino acid sequence between LH and the 1-110 amino acid sequence of the beta subunit of HCG. Accordingly, when it is desired to form an HCG-derived linear polymeric polypeptide of the invention, the fragment used is preferably one having a molecular structure similar to part or all of the 111-145 sequence of the beta subunit of HCG, since it is only this 111-145 sequence of beta-HCG wheih differs from the corresponding sequence of LH. However, research indicates that the fragments used in the linear polymeric polypeptides of the invention may contain sequences corresponding to the 101-110 sequence of beta-HCG which is common to beta-HCG and LH without inducing the formation of antibodies reactive to LH. Thus, one can use in the linear polymeric polypeptides of the invention fragments containing part of or all of this common 101-110 sequence without causing cross-reactivity with LH. For example, Structure (II) above represents the 111-145 amino acid sequence of beta-HCG. If desired, a fragment having the 101-145 amino acid sequence of beta-HCG could be substituted for the fragment of Structure (II) in the linear polymeric polypeptides of the invention without substantially effecting the activity of the linear polymers and without causing cross-reactivity with LH.

Polymerization of the fragments to form the linear polymeric polypeptides of the invention may be effected in any manner for coupling peptide fragments to form linear polymers thereof known to those skilled in the art. The linear polymeric polypeptides of the invention may be divided into two distinct types. In the first type, the individual peptide fragments are linked head-to-tail by peptide linkages, so that the whole polymer comprises solely the fragments themselves and does not

contain any extraneous material. Although such pure polymers do have the advantage of not introducing any extraneous material into the body of the animal being treated, they are usually too expensive to be practical, since the necesary fragments (whether produced by total synthesis or cleavage of a natural protein) are themselves very expensive and substantial losses occur during the polymerization process. Furthermore, the head-to-tail coupling of the fragments, without any intervening residues, may produce immunological determinants which have no counterpart in the unpolymerized fragment. For example, if the fragment described above, comprising the 105-145 sequence of HCG, is polymerized by means of peptide linkages, a sequence:

Pro-Ile-Leu-Pro-Gln-Asp-His-Pro-Leu-Thr

will be produced at each junction between adjacent fragments, and this sequence may provoke the formation of antibodies which would not be produced by the fragment itself, and which may be undesirable. In colloquial terms, since there is no "punctuation" to tell the immune system of the recipient animal where one fragment begins and another ends, the animal's immune system may inadvertently start reading at the wrong residue and produce unwanted antibodies by running the sequences of adjacent fragments together. For this reason, in general, we do not recommend the use of linear polymers in which the fragments are connected by peptide polymers, though of course such linear polymers may be useful in certain instances.

Various methods of coupling polypeptide fragments via peptide bonds are known to those skilled in the art. For example, one fragment to be coupled may have its C-terminal carboxyl group blocked (e.g. by astearification) and be reacted with the other fragment, which has its N-terminal amino group blocked, but its carboxyl group activated by means of an activating agent. Obviously, blocking of non-terminal amino and carboxyl groups may be necessary. Also, as well know to those skilled in this field, it may be advantageous to attached one end of the polymer being produced to a support, such as polystyrine resin support, the polymer only being detached from the support after polymerization is completed.

In the second type of linear polymer polypeptide of theinvention, the polypeptide fragments are connected to one another by means of residues derived from a bifunctional reagent used to effect polymerization of the fragments, so that the final linear polymer is an alternating linear polymer of polypeptide fragments and coupling reagent residues. Although this type of polymer necessarily introduces some extraneous material into the animal being treated, the proportion of extraneous material can be made considerably lower than it would be if the fragments were coupled to a large carrier, such as diphtheria toxoid. The coupling reagent, which is necessarily a bifunctional coupling reagent to produce a true linear polymer, can be chosen so that the resides it leaves in the polymer are not strongly immunogenic (so that they do not place the strain on the immune system of the recipient animal that, for example, a large carrier molecule such as diphtheria toxoid would) and the presence of these residues in the polymer has the advantage of substantially eliminating false immunological determinants produced by conjunction of the head of one fragment with the tail of an adjacent fragment, as discussed above.

To ensure that a true linear polymer is produced during the polymerization process, one terminal of a first polypeptide fragment is reacted with the bifunctional coupling reagent so that the coupling reagent reacts with a group present at or adjacent one terminal of the fragment; for example, the coupling reagent may react with a N-terminal amino group, a C-terminal carboxyl group or a free thiol group present on a C-terminal cysteine. Obviously, the nature of the coupling reagent used determines what group on the peptide reacts. In order to avoid any cross-linking and to ensure a reproduceable product, it is important that only one site on the first fragment be available for reaction with the coupling reagent so that the coupling reagent can only attach to the first fragment at this one site. As those skilled in this field are aware, if it is desired to use a fragment containing more than one group which could react with the coupling reagent, the excess sites may be blocked by attaching suitable protective groups thereto. The product formed by reaction of the first fragment with the coupling reagent is then reacted with a second fragment (which may be the same or different from the first fragment) having a single site available to react with the second reactive group of the bifunctional bicoupling reagent, thereby coupling the first and second fragments by a residue derived from the coupling reagent. Following any necessary purification of this dimeric product, it is then reacted with a further portion of a coupling agent which may be the same or different reagent from that used to effect the first coupling), thereby reacting the free terminal of either the first or second fragment with the coupling reagent. Naturally, it is important to ensure that only one site on the dimer is available for coupling to the coupling reagent, and as will be apparent to those skilled in the art, blocking or unblocking of potential reactive groups on the dimeric polypeptide may be necessary. The product of the reaction of the dimeric polypeptide with the coupling reagent is then reacted with a third fragment having

only a single site available for reaction with the remaining reactive group of the coupling reagent, thereby producing a linear polymer containing three polypeptide fragments. Obviously, this process can be repeated until the desired size of linear polymer has been produced.

It will be apparent to those skilled in this field that the bifunctional coupling reagents used to prepare the linear polymeric polypeptides of the invention should be asymmetric i.e. they should have two functional groups which react with different groups on the fragments being polymerized, since, for example, if one attempted to react a bifunctional bicoupling reagent having two functional groups which both reacted with amino groups with a first fragment having a single amino group, at least some of the first fragment would be dimerized via a residue derived from the bifunctional bicoupling reagent. Such dimerization may in theory be avoided by using a very large excess of the coupling reagent, but in practice it is undesirable to run the risk of producing even a small proportion of dimer. Similarly, in later stages of the polymerization process, it will be even more undesirable to use symmetric coupling reagents, thereby running the risk of dimerizing the partially formed polymers already produced. In the preferred process for producing the linear polymeric polypeptides in the invention already described, the polymer chain is begun with a first peptide having no unblocked thiol group and having an unblocked amino group only at its N-terminal (peptides containing thiol groups and/or amino groups other than at the N-terminal may of course be used if all these thiol and amino groups are blocked with any conventional blocking agent). This first peptide is then reacted with an amino group activating agent, a preferred activating agent for this purpose being 6-maleimido caproic acyl N-hydroxy succinimide ester (MCS); reaction of the peptide with this reagent is optimally effected at a pH of 6.6). The activating agent reacts with the amino group at the N-terminal of the first peptide to form an activated form of the first peptide; in the case of MCS, it is the ester portion of the reagent which reacts with the N-terminal group of the peptide. It is normally then necessary to remove excess activating agent before continuing the preparative process. Once the excess activating agent is removed, the activated first peptide is reacted with a second peptide having a C-terminal cysteine in a reduced state (i.e. having an unblocked 3-thiol group), thereby causing coupling of the N-terminal of the activated first peptide to the C-terminal of the second peptidevia an activating agent residue. Desirably, the resultant dimer is purified as described in more detail below. Next, the dimer is again reacted with an amino-group activating agent and then with a second portion of the second peptide or with

a third peptide, thereby producing a trimer. This procedure is repeated until the desired chain length has been achieved.

In order to secure reproduceable responses from the immune systems of treated animals, it is important that the linear polymeric polypeptides of the invention be used in the form pure polymers in which all the molecules contain the same number of fragments. To achieve such pure polymers, effective purification should be used after each polymerization step of the polymerization process. Because of the close chemical similarity between polymers containing different numbers of fragments, chemical purification is ineffective, so purification must be effected by physical methods. Gel filtration may be used if desired, but our preferred purification method is reverse-phase, high-pressure liquid chromatography, preferably using a molecular sieve as the solid phase.

In this method of forming linear polymers, the first and second peptides may be identical in chemical configuration except that in the first peptide the C-terminal cysteine has a blocked thiol group. The first two preferred fragments for forming linear polymeric polypeptides of the invention to form antibodies to HCG mentioned above may be described as (111-145)-Cys and (105-145)-Cys, where the figures refer to the amino acid sequence in the beta subunit of HCG. It will be appreciated that, when these fragments are to be used in forming linear polymeric polypeptides of the invention by the method just described, the lysine residue at position 122 must have its amino group blocked and, in the case of the (105-145)-Cys fragment, the non-terminal cysteine at position 110 must have its thiol group blocked, preferably with an acetamidomethyl group.

The linear polymeric peptides of the invention preferably contain from about 4 to about 14 fragments.

The modified antigens of the invention produced by chemically modifying non-endogenous proteins will now be described in more detail. As those skilled in the art are aware, there are numerous pathogens and similar materials known which are not endogenous to animals, which are capable of producing harmful effects in the animal's body but which are not immunogenic to the animal, in the sense that introduction of the pathogen or other material into the body of the animal fails to elicit from the animal's immune system production of the quantity of appropriate antibodies necessary for the animal's immune system to destroy the pathogen or similar material. For example, the Herpes simplex Type II virus is capable of producing a number of harmful effects in man, including the production of painful lesions in the genital areas. Although this virus has, like most viruses, protein

included in its structure, the viral protein is not strongly immunogenic in most human beings, so that only about 50% of infected human beings produce antibodies to the virus. This lack of immune response to the virus by many human beings means that the virus can remain in the infected human beings for at least several years, and this persistence of the virus in the infected individuals not only causes those individuals to suffer recurrent attacks of the painful symptoms caused by the virus, but also renders them long-term carriers of the virus. This persistence of the virus in infected individuals is one of the factors largely responsible for the epidemic proportions which Herpes simplex infections have reached in several countries. By preparing an antigen of this invention derived from a protein having a sequence similar to that of at least part of the sequence of a Herpes simplex viral protein, it is possible to stimulate the human immune system so as to render it capable of producing large quantities of antibodies to the Herpes simplex virus. Not only should this stimulation of the immune system reduce the occurrence of symptoms associated with Herpes simplex infection, but it should help to control the spread of the virus. Similarly, the immune response of humans and other animals to viruses such as colds, influenza and other viruses can be increased by preparing modified antigens of the invention based upon peptides having sequences corresponding to viral proteins of the appropriate virus. If, as appears likely, a virus is responsible for acquired immune deficiency syndrome (AIDS) a modified antigen of this invention could also be used to produce immunity to this disease.

In general, the methods used for preparing the antigens of the invention based upon non-endogenous materials, such as viral proteins or peptides corresponding to parts thereof, are the same as those used for modifying endogenous proteins or fragments thereof, as described in our aforementioned U.S. patent No. 4,302,386. However, it will be appreciated that the preferred methods used for modifying non-endogenous materials may differ in certain respects from those used for modifying endogenous peptides. Since, in general, the non-endogenous peptide will provoke at least a limited immune response from the animal to which the antigen is to be administered, the requirements for modification of the non-endogenous protein or peptide to produce an antigen of the invention will tend to be less stringent than those for modification of an endogenous and completely non-immunogenic protein or peptide. However, since the non-endogenous protein or peptide is being modified to increase its immunogenic effect in the animal into which it is to be administered, in general it will still be desirable that the carrier used to modify the non-endogenous protein or peptide (if chemical modification is to be effected by coupling the non-endogenous protein or peptide to a carrier, rather forming a linear polymeric polypeptide of the invention) to produce the antigen of the invention be a material which itself provokes a strong response from the animal's immune system. For example, the carrier may be a bacterial toxoid such as diphtheria toxoid or tetanus toxoid.

The antigens of the invention are prepared by chemically modifying a protein which is not endogenous or immunogenic to the animal to be treated, or a peptide having a sequence corresponding to at least part of the sequence of the protein, this chemical modification being effected outside the body of the animal. The chemical modification may be effected by coupling the protein or peptide to a carrier, by polymerizing the peptide to form a linear polymeric polypeptide of the invention or by any other chemical modification technique which will make the protein or peptide sufficiently immunogenic. Preferred techniques for chemical modification of the protein or peptide by coupling to a carrier include the following (further details of optimum techniques for each of the following coupling reactions are given in our aforementioned U.S. patent No. 4,302,386):

a. Treating a protein or peptide having a sulfhydryl group thereon with an activator of the structure:

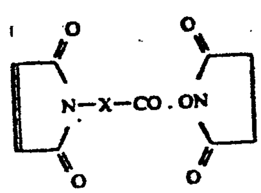

or

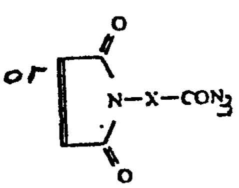

wherein X represents a non-reacting group comprising substitued or unsubstituted phenyl or $C_1$-10 alkylene moieties, or a combination thereof, or an amino acid chain, so as to cause reaction of the maleiimide group of the activator with the sulfhydryl group on the protein or peptide; and treating the resulting activated protein or peptide at slightly alkaline pH with a carrier moiety biologically foreign to the animal and selected having a size sufficient to elicit antibody response following the administration thereof into the body of the animal.

b. Treating, under neutral or acid conditions, a carrier not having a sulfhydryl group but having an amino group with an activator as defined in the proceding paragraph, so as to cause reaction of the activator with

the amino group on the carrier, the carrier being biologically foreign to the animal to be treated and having a size sufficient to elicit antibody response following administration thereof into the body of the animal; and treating the resulting activated carrier with the protein or peptide, which must have a sulfhydryl group thereon, thereby reacting the maleiimide group of the activator with the sulfhydryl group on the protein or peptide.

c. Treating a carrier biologically foreign to the animal to be treated and having an amino group with an activator present as an active ester of chloro-, dichloro-, bromo- or iodo- acetic acid so as to cause reaction of the activator with the amino group of the carrier and treating the resulting activated carrier with the protein or peptide, which must have a sulfhydryl group thereon, thereby reacting the activated carrier with the sulfhydryl group of the protein or peptide.

d. Treating a protein or peptide that does not have a sulfhydryl group but has an amino group with an activator present as an active ester of chlor-, dichloro-, bromo- or iodo- acetic acid, and treating the resulting halomethyl alkylating group containing moiety with a sulfhydryl group-containing carrier biologically foreign to the animal to be treated so as to react the sulfhydryl group of the carrier with the halomethyl alkylating group.

It will be noted that several of the preferred coupling techniques described above require the presence of a sulfhydryl group in the protein or peptide.

It is well known to those skilled in the art that, in many natural proteins containing cysteine residues, these residues are not present in their thio form containing a free-SH group; instead pairs of cysteine residues are lined by means of disulfide bridges to form cystine. Such disulfide bridges are very important in determining the conformation of the protein. In most cases, the disulfide bridges present in the natural form of the protein are easily reduced to pairs of -SH groups by means of mild reducing agents under conditions which leave the remaining parts of the protein molecule unchanged. Accordingly, when it is desired to produce free -SH groups in proteins in order to carry out the coupling reaction sdiscussed above, one convenient way of providing such free-SH groups may be to cleave disulfide bridges naturally present in the protein or other polypeptide which is desired to couple.

The generation of free-SH by reduction of disulfide bridges in naturally occuring forms of proteins may also affect the cross-reactivity of the antibodies produced when a modified polypeptide produced from the protein is injected into an animal. Frequently, an antibody recognizes its corresponding antigen not only by the amino acid sequence in the antigen but also by the conformation (shape) of the antigen. Accordingly, an antibody which binds very strongly to a protein or other polypeptide in its natural conformation may bind much less strongly, if at all, to the same protein or polypeptide whose conformation has been drastically altered by breaking disulfide bridges therein. Accordingly, the breaking of disulfide bridges in proteins or other polypeptides may provide a basis for reducing the cross-reactivity between antibodies to antigens having the same amino acid sequence along parts of the molecule.

As mentioned above, this invention extends to chemically modified antigens derived from zona pellucida or sperm antigens, or peptides having a sequence corresponding to at least part of the sequence of such a zona pellucida or sperm antigen. These modified antigens of the invention are useful for fertility control. It is known that antigens from the zona pellucida (the outer covering of the ovum) when injected into female primates produce antibodies having anti-fertilization effects, including prevention of sperm attachment to, and penetration of, the zona pellucida of the unfertilized ovum, and prevention of dispersal of the zona pellucida of the fertilized ovum prior to implantation (such dispersal of the zona apparently being an essential prerequisite for implantation). See e.g. W.R. Jones, "Immunological Fertility Regulation", Blackwell Scientific Publications, Victoria, Australia (1982), pages 160 et seq. Such anti-fertility effects are believed to be due to formation of an antibody-antigen precipitate on the zona, this precipitate rendering the zona unable to undergo its normal sperm-binding reaction and also rendering the zona insensitive to the action of the protreases normally responsible for dispersal of the zona.

An anti-fertility vaccine based upon zona antigens is especially attractive because zona antigens appear to be relatively free of side-effects on other tissues and because methods have been developed for producing swine zona antigens in large quantities; swine and human zona antigens show very good cross-reactivity. As in the case of anti-fertility vaccines based upon the beta-HCG antigens discussed above and in our aforementioned U.S. patent No. 4,302,386, better results will be obtained by modifying a zona antigen or a fragment thereof to produce a modified antigen of this invention.

Several antigens, especially sperm enzymes, known to exist in sperm, may be used in the modified antigens of this invention; see W.R. Jones, op. cit., pages 133 et seq. The most promising such antigen is the lactate hydrogenase known as LDH-C4 or LDH-X. Although of course lactate dehydrogenases are present in other tissues, LDH-C4 is distinct from other lactate dehydrogenase isoenzymes and appears to be sperm-specific. Moreover, the enzyme is not strongly species specific, and methods for its isolation and purification are known. Again, the best results will be obtained by modifying LDH-C4 or a fragment thereof to produce a modified polypeptide of this invention. Several natural peptide fragments of LDH-C4 have been prepared, sequenced and shown to bind to antibodies against the parent molecule. (See E. Goldburg, "LDH-X as a sperm-specific antigen", in T. Wegmann and T.J. Gill (eds.), Reproductive Immunology, Oxford University Press, 1981). (The disclosure of this work is herein incorporated by reference.)

Although theoretically an anti-fertility vaccine based on sperm antigens might be useful in males, the likelihood of testicular damage renders it more likely that such a vaccine will find its utility in females. It is known that circulating antibodies in the female bloodstream do penetrate the genital fluids; for example experiments in baboons with vaccines based upon the peptide of Structure (XII) above conjugated with tetanus toxoid have shown the presence of HCG antibodies in the genital fluids. However, one possible problem with any vaccine based on sperm antigens is maintaining a sufficiently high antibody level in female genital fluids to complex with the large amounts of sperm involved.

The techniques used for chemical modification of zona pellucida or sperm antigens, or peptides derived therefrom, to form the modified antigens of the invention include all the techniques discussed in our aforementioned U.S. patent No. 4,302,386 and also those techniques discussed above. Thus, the antigen or peptide may be coupled to a carrier, such as tetanus toxoid or diphtheria toxoid, or may be polymerized to form a linear polymeric polypeptide of the invention. The preferred techniques for forming such linear polymeric polypeptides have already been discussed above, while the preferred techniques for coupling the antigens or peptides to carriers are the same as those for coupling non-endogenous proteins or peptides, as already discussed above.

Finally, as mentioned above, this invention extends to a vaccine containing a modified polypeptide, antigen or modified antigen of the invention and a vehicle comprising a mixture of mannide monooleate with squalane and/or squalene. It has been found that this vehicle has the effect of increasing the quantity of antibodies

provoked by the linear polymeric polypeptide, antigen or modified antigen of the invention when the vaccine is administered to an animal. To further increase the quantity of antibodies provoked by administration of the vaccine, it is advantageous to include in the vaccine an immunological adjuvant. The term "adjuvant" is used in its normal meaning to one skilled in the art of immunology, namely as meaning a substance which will elevate the total immune response of the animal to which the vaccine is administered i.e. the adjuvant is a non-specific immuno-stimulator. Preferred adjuvants are muramyl dipeptides, especially:

NAc-nor Mur-L.Ala-D.isoGln;
NAc-Mur-(6-0-stearoyl)-L.Ala-D.isoGln; or
NGlycol-Mur-L.αAbu-D.isoGln

The vaccines of this invention may be administered parenterally to the animals to be protected; the usual modes of administration of the vaccine being intramuscular and sub-cutaneous injections. The quantity of vaccine to be employed will of course vary depending upon various factors, including the condition being treated and its severity. However, in general, unit doses of 0.1-50 mg. in large mammals administered with one to five times the intervals of 1 to 5 weeks provide satisfactory results. Primary immunization may also be followed by "booster" immunization at 1 to 12 month intervals.

To prepare the vaccines of the invention, it is convenient to first mix the linear polymeric polypeptide, antigen or modified antigen of the invention with the muramyl dipeptide (or other adjuvant) and then to emulsify the resultant mixture in the mannide monooleate/squalene or squalane vehicle. Squalene is preferred to squalane for use in the vaccines of the invention, and preferably about 4 parts by volume of squalene and/or squalane are used per part by volume of mannide monooleate.

The animals to be treated with the linear polymeric polypeptides, antigens, modified antigens and vaccines of this invention include both humans and other animal species.

The following example is now given, though by way of illustration only, to show details of the preparation and use of a linear polymeric polypeptide of the invention.

## EXAMPLE

Fragment A described above (a fragment having an amino acid sequence corresponding to the 105-145 sequence of beta-HCG, with a cysteine residue added to the C-terminal thereof) was polymerized to form a hexamer. A first portion of

fragment A had both its thiol groups (on the non-terminal cysteine at the position corresponding to position 110 of beta-HCG, and on its C-terminal cysteine) and its non-terminal amino group (on the lysine residue at the position corresponding to position 122 in beta-HCG) blocked. This blocked form of Fragment A was reacted with the bifunctional organic coupling reagent (or amino group activating agent) MCS in a buffered aqueous solution at pH 6.6, thereby reacting the ester portion of the MCS with the N-terminal amino group of the first portion of Fragment A. The resultant product was then reacted with a second portion of Fragment A, which was used in the same form as the first portion of Fragment A except that the C-terminal cysteine bore an unblocked thiol group, thereby reacting the remaining functional group of the MCS with the free thiol group on the second portion of Fragment A and producing a dimer in which the N-terminal of the first portion of Fragment A was coupled to the C-terminal of the second portion of Fragment A via an MCS residue. This dimer was then purified by gel filtration. The polymerization was then repeated in the same manner until a hexamer of Fragment A had been produced. Because the purification following each polymerization step was effected by gel filtration rather than by reverse-phase, high-pressure liquid chromatography, the hexamer was undoubtedly somewhat impure and contaminated by traces of pentamer, tetramer etc., so that the results in the animal tests results described below could not be expected to be as good as would be produced using a pure hexamer.

To test the effectiveness of this hexameric polypeptide in provoking the formation of antibodies to HCG, the hexamer was formed into a vaccine using Complete Freunds' Adjuvant and injected into five rabbits. Each rabbit was given three injections of the vaccine intramuscularly at 3 week intervals, each injection containing 0.5 mg. of the hexamer. Starting three weeks after the first injection, each rabbit was bled weekly and the leverl of antibodies to HCG in the blood determined. The following average values of antibody level were found (the figures in parenthesis represent the confidence limits i.e. average + or - standard error):

TABLE

| Weeks After First Injection | Antibody concentration (moles/liters x $10^{-10}$) |
|---|---|
| 3 | 5 (2-7) |
| 4 | 18 (13-22) |
| 5 | 30 (21-39) |
| 6 | 45 (30-60) |
| 7 | 58 (32-83) |
| 8 | 61 (34-86) |
| 9 | 77 (50-108) |
| 10 | 110 (53->125) |
| 11 | 100 (50->125) |
| 12 | 79 (30-119) |
| 13 | 53 (22-83) |

The, the above results show that even the crude hexamer preparation used in these experiments was much more strongly immunogenic than the very weakly immunogenic fragment from which it was derived.

## CLAIMS

1. A modified antigen for use in fertility control in an animal, characterized in that it comprises an antigen derived from the zona pellucida or from sperm, or a peptide having a sequence corresponding to at least part of the sequence of such a zona pellucida or sperm antigen, which antigen or peptide has been chemically modified outside the body of the animal, the modified antigen, after administration into the body of the animal, having a greater capacity to provoke the formation of antibodies than the unmodified antigen from which it is derived.

2. A modified antigen according to claim 1 characterized in that it comprises a plurality of the peptides connected to one another via residues of a bifunctional reagent used to couple the peptides together.

3. A modified antigen according to claim 1 characterized in that it comprises a plurality of the peptides connected to one another by peptide linkages without any intervening residues.

4. A modified antigen according to claim 1 characterized in that it comprises a plurality of the peptides connected to one another by a chemical modification technique comprising:

(a) reacting a first one of the peptides with a bifunctional coupling reagent while the first peptide is in a form having only a single site capable of reacting with the coupling reagent, this site being at or adjacent one of the termini of the first peptide, thereby causing one of the functional groups of the coupling reagent to react with the one site on the peptide;

(b) reacting the product of step (a) with a second one of the peptides while the second peptide is in a form having only a single site capable of reacting with the other functional group of the coupling reagent, this site being at or adjacent one of the termini of the second peptide, thereby forming a dimeric peptide wherein the first and second peptides are interconnected via a residue of the coupling reagent;

(c) reacting the resultant peptide with a bifunctional coupling reagent while the peptide is in a form having only a single site capable of reacting with the coupling reagent, this site being at or adjacent one of the termini of the peptide, thereby causing one of the functional groups of the coupling reagent to react with the one site on the peptide;

(d) reacting the product of step (c) with a further one of the peptides while the further peptide is in a form having only a single site capable of reacting

with the other functional group of the coupling reagent, this site being at or adjacent one of the termini of the further peptide, thereby forming a polymeric peptide wherein the first, second and further peptides are interconnected via residues of the coupling reagent; and

(e) repeating steps (c) and (d) until the desired polymer length has been achieved.

5. A modified antigen according to claim 1 characterized in that it comprises a plurality of the peptides connected to one another by a chemical modification technique comprising:

(a) reacting a first one of the peptides, this first peptide not having an unblocked thiol group and having an unblocked amino group at its N-terminal but no other unblocked amino group, with an amino group activating agent, thereby producing an activated amino group at the N-terminal of the first peptide;

(b) reacting the activated first peptide with a second one of the peptides, the second peptide having a C-terminal cysteine bearing an unblocked thiol group but not having any other unblocked thiol groups, thereby coupling the N-terminal of the first peptide to the C-terminal of the second peptide;

(c) reacting the resultant compound, in a form having an unblocked amino group at its N-terminal but no other unblocked amino groups, and no unblocked thiol

groups, with an amino group activating agent, thereby producing an activated amino group at the N-terminal of the resultant compound;

(d) reacting the activated compound produced in step (c) with a further one of the peptides, the further peptide having a C-terminal cysteine bearing an unblocked thiol group but not having any other unblocked thiol groups, thereby coupling the activated N-terminal of the reactivated compound produced in step (c) to the C-terminal of the further peptide; and

(e) repeating steps (c) and (d) until the desired polymer length has been achieved.

6. A modified antigen according to claim 1 characterized in that the chemical modification has been effected by a process comprising:

treating the antigen or peptide, which has a sulfhydryl group thereon, with an activator of the structure

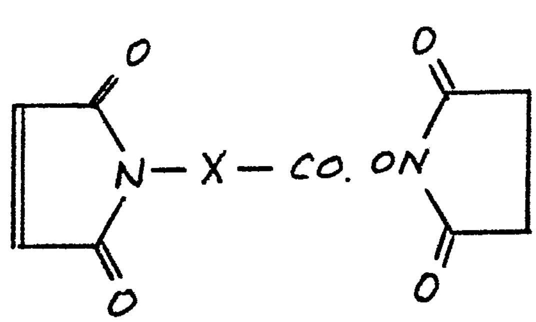

or

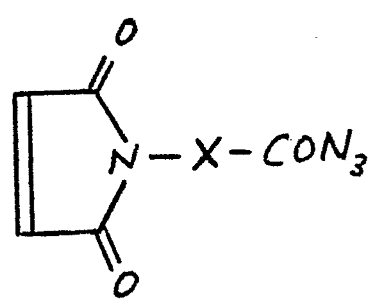

wherein X represents a non-reacting group comprising substituted or unsubstituted phenyl or $C_{1-10}$ alkylene moieties, or a combination thereof, or an

amino acid chain, so as to cause reaction of the maleimide group of the activator with the sulfhydryl group on the antigen or peptide; and

treating the resulting activated antigen or peptide at slightly alkaline pH with a carrier moiety biologically foreign to the animal and selected having a size sufficient to elicit antibody response following the administration thereof into the body of the animal, thereby producing the modified antigen.

7. A modified antigen according to claim 1 characterized in that the chemical modification has been effected by a process comprising:

procuring a carrier which is biologically foreign to the animal, has a size sufficient to elicit antibody response following the administration thereof into the body of the animal and does not have a sulfhydryl group but has an amino group;

treating the carrier under neutral or acid conditions with an activator of the structure

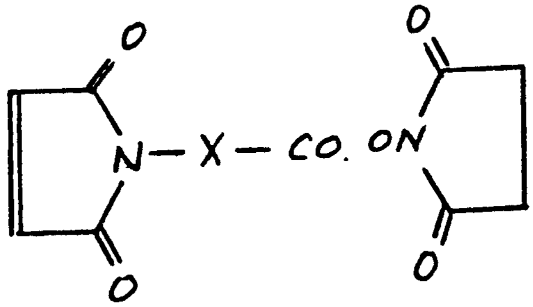

or

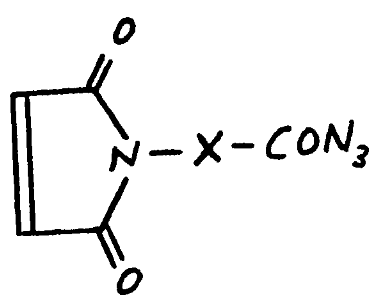

wherein X represents a non-reacting group comprising substituted or unsubstituted phenyl or $C_{1-10}$ alkylene moieties, or a combination thereof, or an amino acid chain, so as to cause reaction of the activator with the amino group on the carrier; and

treating the resultant activated carrier with the antigen or peptide, the antigen or peptide having a sulfhydryl group thereon, thereby reacting the maleiimide group of the activator with the sulfhydryl group on the antigen or peptide and producing the modified antigen.

8. A modified antigen according to claim 1 characterized in that the chemical modification has been effected by a process comprising:

procuring a carrier which is biologically foreign to the animal, has a size sufficient to elicit antibody response following the administration thereof into the body of the animal and does not have a sulfhydryl group but has an amino group;

treating the carrier with an activator present as an active ester of chloro-, dichloro-, bromo- or iodo-acetic acid so as to cause reaction with the amino group on the carrier; and

treating the resultant activated carrier with the antigen or peptide, the antigen or peptide having a sulfhydryl group thereon, thereby reacting the activated

carrier with the sulfhydryl group on the antigen or peptide and producing the modified antigen.

9. A modified antigen according to claim 1 characterized in that the chemical modification has been effected by a process comprising:

treating the antigen or peptide, which does not have a sulfhydryl group but which has an amino group thereon, with an activator present as an active ester of chloro-, dichloro-, bromo- or iodo-acetic acid, thereby reacting the active ester with the amino group on the antigen or peptide to produce a halomethyl alkylating group containing moiety; and

treating the halomethyl alkylating group containing moiety with a carrier which is biologically foreign to the animal, has a size sufficient to elicit antibody response following the administration thereof into the body of the animal and contains a sulfhydryl group, thereby reacting the sulfhydryl group with the halomethyl alkylating group to form the modified antigen.

10. A vaccine for provoking the formation, in the body of an animal, of antibodies to a protein, the vaccine being characterized in that it comprises a modified antigen prepared according to any one of the preceding claims and a vehicle comprising a mixture of mannide monooleate with Squalane and/or Squalene.